(19)

Europäisches
Patentamt
European
Patent Office
Office européen
des brevets

(11) **EP 2 609 869 A1**

(12) **EUROPEAN PATENT APPLICATION**

(43) Date of publication:
**03.07.2013 Bulletin 2013/27**

(51) Int Cl.:
*A61B 8/06* (2006.01)          *G01S 15/89* (2006.01)

(21) Application number: **12199547.6**

(22) Date of filing: **27.12.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.12.2011 KR 20110144461**

(71) Applicant: **Samsung Medison Co., Ltd.
Gangwon-do 250-870 (KR)**

(72) Inventor: **Kim, Hyoung Jin
135-851 Seoul (KR)**

(74) Representative: **Lorenz, Markus
Lorenz & Kollegen
Patentanwälte Partnerschaftsgesellschaft
Alte Ulmer Strasse 2
89522 Heidenheim (DE)**

(54) **Providing particle flow image in ultrasound system**

(57)     There are provided embodiments for providing
a particle flow image for representing flow of at least one
region of interest (more particularly, particle) by using
vector Doppler. In one embodiment, by way of non-limiting example, an ultrasound system comprises: a
processing unit configured to form vector information of
a target object based on ultrasound data corresponding
to the target object, form a Doppler mode image based
on the vector information, and set at least one region of
interest on the Doppler mode image based on input information of a user, the processing unit being further configured to form a flow image for representing flow of the
at least one region of interest based on the vector infor-
mation.

## FIG. 15

START

FORMING BRIGHTNESS MODE IMAGE — S1502

SETTING REGION OF INTEREST — S1504

FORMING VECTOR INFORMATION — S1506

FORMING DOPPLER MODE IMAGE — S1508

SETTING PARTICLE — S1510

FORMING PARTICLE FLOW IMAGE — S1512

END

EP 2 609 869 A1

Printed by Jouve, 75001 PARIS (FR)

**Description**

CROSS-REFERENCE TO RELATED APPLICATIONS

The present application claims priority from Korean Patent Application No. 10-2011-0144461 filed on December 28, 2011.

TECHNICAL FIELD

The present disclosure generally relates to ultrasound systems, and more particularly to providing a particle flow image for representing flow of at least one region of interest (more particularly, particle) by using vector Doppler in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional or three-dimensional ultrasound images of internal features of target objects (e.g., human organs).

The ultrasound system provides ultrasound images of various modes including a brightness mode image representing reflection coefficients of ultrasound signals (i.e., ultrasound echo signals) reflected from a target object of a living body with a two-dimensional image, a Doppler mode image representing velocity of a moving target object with spectral Doppler by using a Doppler effect, a color Doppler mode image representing velocity of the moving target object with colors by using the Doppler effect, an elastic image representing mechanical characteristics of tissues before and after applying compression thereto, and the like.

The ultrasound system transmits the ultrasound signals to the living body and receives the ultrasound echo signals from the living body to form Doppler signals corresponding to a region of interest, which is set on the brightness mode image. The ultrasound system further forms the color Doppler mode image representing the velocity of the moving target object with colors based on the Doppler signals. In particular, the color Doppler image represents the motion of the target object (e.g., blood flow) with the colors. The color Doppler image is helpfully used to diagnose disease of a blood vessel, a heart and the like. However, it is difficult to represent a accurate motion of the target object (e.g., blood flow) since the respective colors indicated by a motion value is a function of the velocity of the target object which moves forward in a transmitting direction of the ultrasound signals and moves backward in the transmitting direction of the ultrasound signals.

To resolve this problem, a vector Doppler method capable of obtaining a velocity and a direction of the blood flow is used. A cross beam-based method of the vector Doppler method acquires velocity magnitude components from at least two different directions, and combine the velocity magnitude components to detect vector information having a two-dimensional or three-dimensional direction information and a magnitude information.

SUMMARY

There are provided embodiments for providing a particle flow image for representing flow of at least one region of interest (more particularly, particle) by using vector Doppler.

In one embodiment, by way of non-limiting example, an ultrasound system comprises: a processing unit configured to form vector information of a target object based on ultrasound data corresponding to the target object, form a Doppler mode image based on the vector information, and set at least one region of interest on the Doppler mode image based on input information of a user, the processing unit being further configured to form a flow image for representing flow of the at least one region of interest based on the vector information.

In another embodiment, there is provided a method of providing a particle flow image, comprising: a) forming vector information of a target object based on ultrasound data corresponding to the target object; b) forming a Doppler mode image based on the vector information; c) setting at least one region of interest on the Doppler mode image based on input information of a user; and d) forming a flow image for representing flow of the at least one region of interest based on the vector information.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.
FIG. 2 is a schematic diagram showing an example of a brightness mode image and a region of interest.
FIG. 3 is a block diagram showing an illustrative embodiment of an ultrasound data acquiring unit.
FIGS. 4 to 7 are schematic diagrams showing examples of transmission directions and reception directions.
FIG. 8 is a schematic diagram showing an example of sampling data and pixels of an ultrasound image.
FIGS. 9 to 12 are schematic diagrams showing examples of performing a receiving beam-forming.
FIG. 13 is a schematic diagram showing an example of setting weights.
FIG. 14 is a schematic diagram showing an example of setting a sampling data set.
FIG. 15 is a flow chart showing a process of forming a motion mode image.
FIG. 16 is a schematic diagram showing an example of the transmission directions, the reception directions, the vector information and an over-determined problem.
FIG. 17 is a schematic diagram showing an example of a second region of interest.

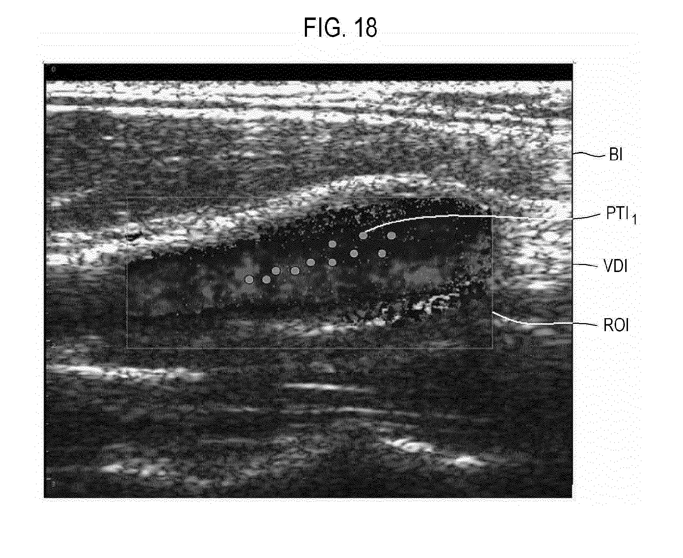

FIGS. 18 and 19 are schematic diagrams showing examples of particle flow images.

DETAILED DESCRIPTION

A detailed description may be provided with reference to the accompanying drawings. One of ordinary skill in the art may realize that the following description is illustrative only and is not in any way limiting.

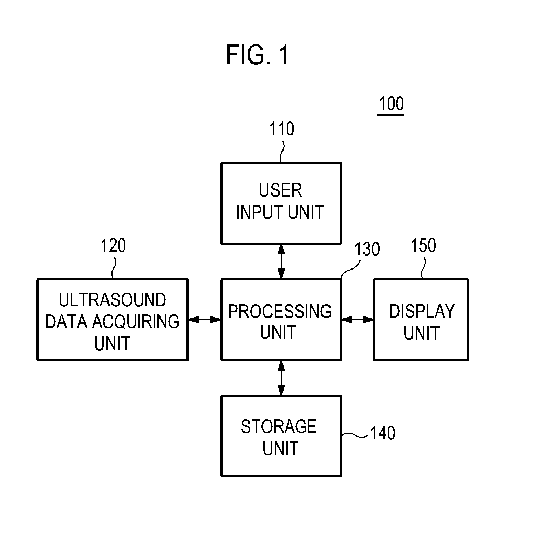

Referring to FIG. 1, an ultrasound system 100 in accordance with an illustrative embodiment is shown. As depicted therein, the ultrasound system 100 includes a user input unit 110.

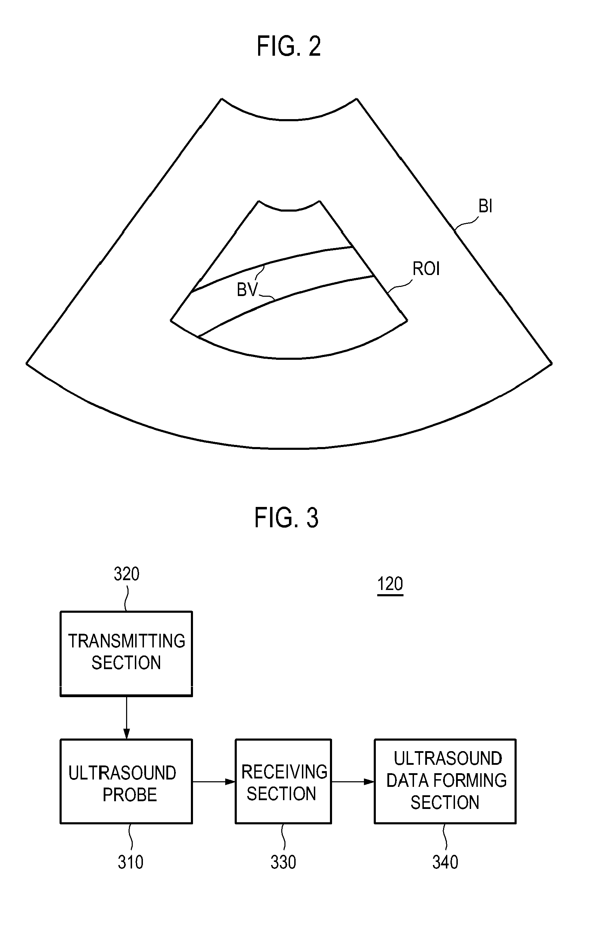

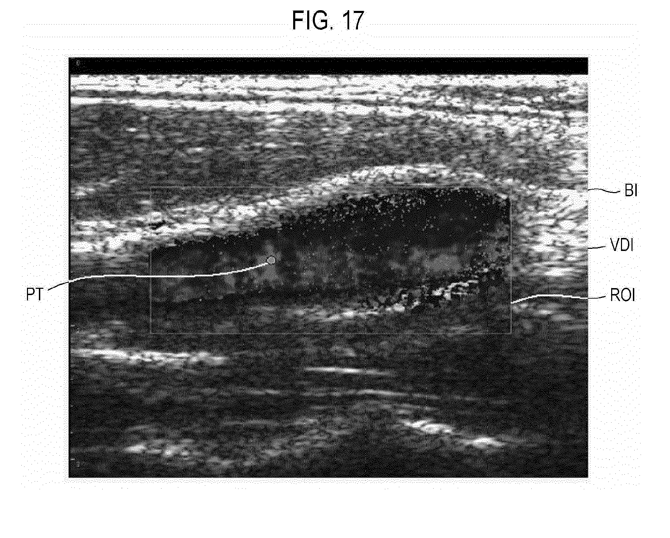

The user input unit 110 is configured to receive input information from a user. In one embodiment, the input information includes first input information for setting a first region of interest ROI on a brightness mode image BI, as shown in FIG. 2. The first region of interest ROI includes a color box for obtaining a Doppler mode image. The Doppler mode image includes a vector Doppler image or a color Doppler image. However, it should be noted herein that the Doppler mode image may not be limited thereto. The input information further includes second input information for setting at least one second region of interest PT on the Doppler mode image VDI, as shown in FIG. 17. The second region of interest PT includes a particle. However it should be noted herein that the second region of interest PT may not be limited thereto. The input information further includes third input information for applying a weighting value to the at least one second region of interest. The weighting value includes weight corresponding to the second region of interest. However, it should be noted herein that the weighting value may not be limited thereto. For example, the weighting value further includes a color, a size and the like. In FIG. 2, a reference numeral BV represents a blood vessel. The user input unit 110 includes a control panel, a track ball, a mouse, a keyboard and the like.

The ultrasound system 100 further includes an ultrasound data acquiring unit 120. The ultrasound data acquiring unit 120 is configured to transmit ultrasound signals to a living body. The living body includes moving target objects (e.g., blood vessel, heart, blood flow, etc). The ultrasound data acquiring unit 120 is further configured to receive ultrasound signals (i.e., ultrasound echo signals) from the living body to acquire ultrasound data corresponding to an ultrasound image.

FIG. 3 is a block diagram showing an illustrative embodiment of the ultrasound data acquiring unit. Referring to FIG. 3, the ultrasound data acquiring unit 120 includes an ultrasound probe 310.

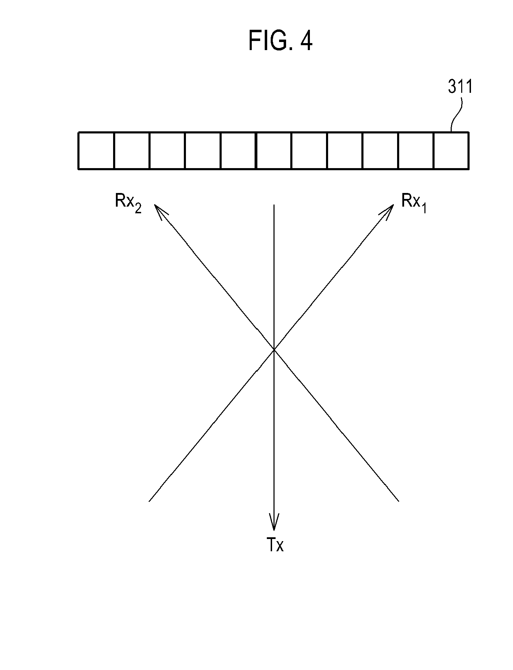

The ultrasound probe 310 includes a plurality of elements 311 (see FIG. 4) for reciprocally converting between ultrasound signals and electrical signals. The ultrasound probe 310 is configured to transmit the ultrasound signals to the living body. The ultrasound signals transmitted from the ultrasound probe 310 are plane wave signals or focused signals. However, it should be noted herein that the ultrasound signals may not be limited thereto. The ultrasound probe 310 is further configured to receive the ultrasound echo signals from the living body to output electrical signals (hereinafter referred to as "reception signals"). The reception signals are analog signals. The ultrasound probe 310 includes a convex probe, a linear probe and the like.

The ultrasound data acquiring unit 120 further includes a transmitting section 320. The transmitting section 320 is configured to control the transmission of the ultrasound signals. The transmitting section 320 is further configured to generate electrical signals (hereinafter referred to as "transmission signals") in consideration of the elements 311.

In one embodiment, the transmitting section 320 is configured to generate transmission signals (hereinafter referred to as "brightness mode transmission signals") for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output reception signals (hereinafter referred to as "brightness mode reception signals").

The transmitting section 320 is further configured to generate transmission signals (hereinafter referred to as "Doppler mode transmission signals") corresponding to an ensemble number in consideration of the elements 311 and at least one transmission direction of the ultrasound signals (i.e., transmission beam). Thus, the ultrasound probe 310 is configured to convert the Doppler mode transmission signals provided from the transmitting section 320 into the ultrasound signals, transmit the ultrasound signals to the living body in the at least one transmission direction, and receive the ultrasound echo signals from the living body to output reception signals (hereinafter referred to as "Doppler mode reception signals"). The ensemble number represents the number of transmitting and receiving the ultrasound signals.

As one example, the transmitting section 320 is configured to generate the Doppler mode transmission signals corresponding to the ensemble number in consideration of a transmission direction Tx and the elements 311, as shown in FIG. 4. The transmission direction is one of a direction (0 degree) perpendicular to a longitudinal direction of the elements 311 to a maximum steering direction of the transmission beam.

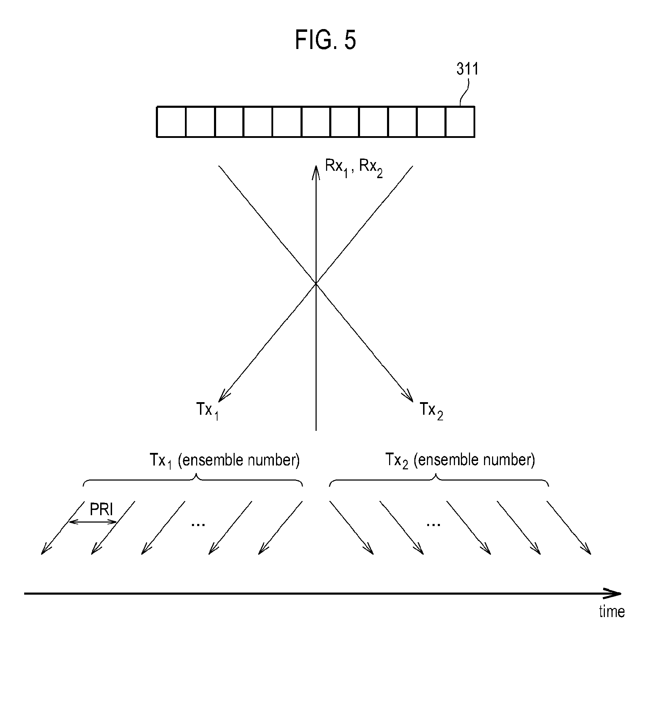

As another example, the transmitting section 320 is configured to generate first Doppler mode transmission signals corresponding to the ensemble number in consideration of a first transmission direction $Tx_1$ and the elements 311, as shown in FIG. 5. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output first Doppler mode reception signals. The transmitting section 320 is further configured to generate second Doppler mode transmission signals corresponding to the ensemble number in consideration of a second transmission direction $Tx_2$ and the elements 311, as shown in FIG. 5. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output second Doppler mode reception signals. In FIG. 5, a reference numeral PRI represents a pulse repeat interval.

In another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted in an interleaved transmission scheme.

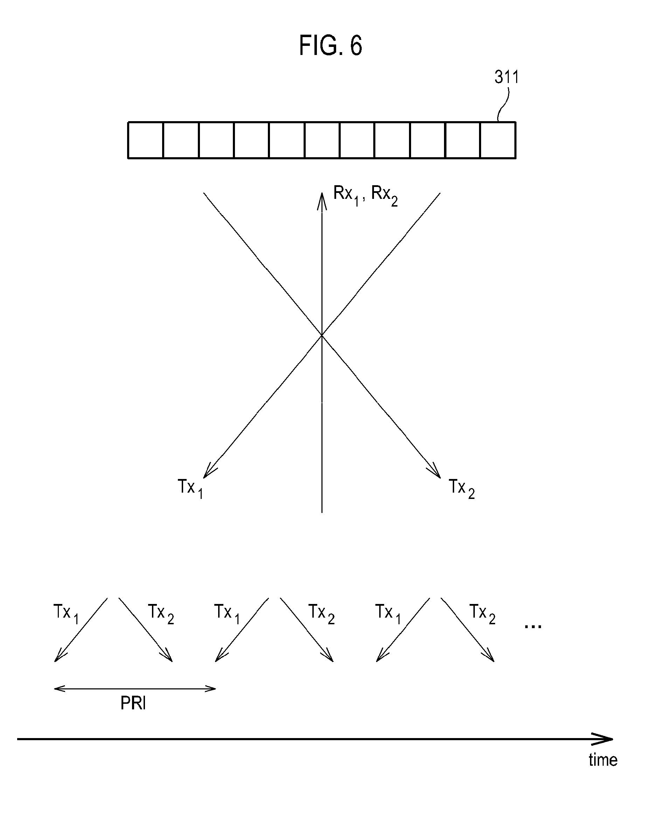

For example, the transmitting section 320 is configured to generate the first Doppler mode transmission signals in consideration of the first transmission direction $Tx_1$ and the elements 311, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. Thereafter, the transmitting section 320 is further configured to generate the second Doppler mode transmission signals in consideration of the second transmission direction $Tx_2$ and the elements 311, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

Thereafter, the transmitting section 320 is configured to generate the first Doppler mode transmission signals based on the pulse repeat interval, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. The transmitting section 320 is further configured to generate the second Doppler mode transmission signals based on the pulse repeat interval, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

As described above, the transmitting section 320 is configured to generate the first Doppler mode transmission signals and the second Doppler mode transmission signals corresponding to the ensemble number.

In yet another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted according to the pulse repeat interval.

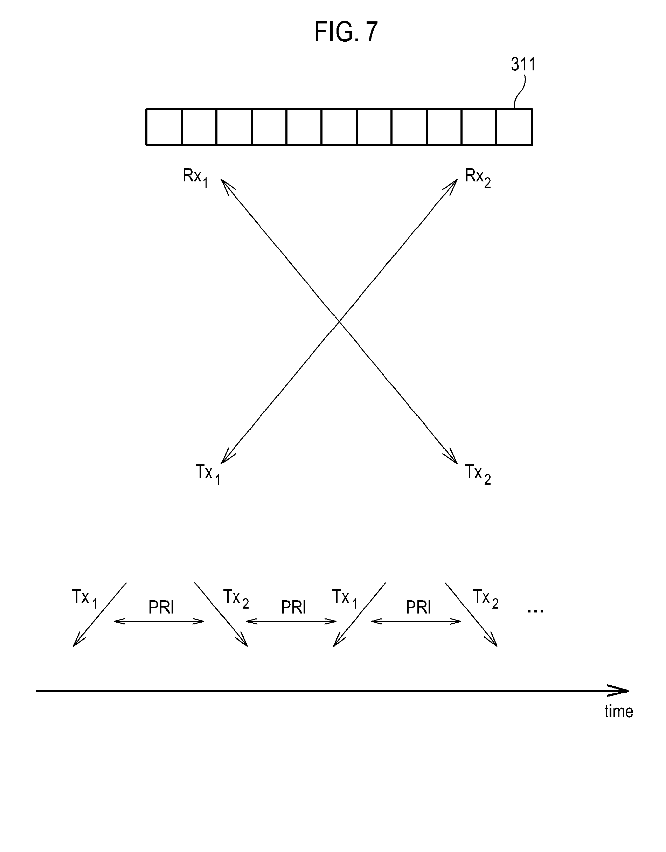

For example, the transmitting section 320 is configured to generate the first Doppler mode transmission signals in consideration of the first transmission direction $Tx_1$ and the elements 311, based on the pulse repeat interval, as shown in FIG. 7. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output the first Doppler mode reception signals. The transmitting section 320 is further configured to generate the second Doppler mode transmission signals in consideration of the second transmission direction $Tx_2$ and the elements 311, based on the pulse repeat interval, as shown in FIG. 7. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output the second Doppler mode reception signals.

As described above, the transmitting section 320 is configured to generate the first Doppler mode transmission signals and the second Doppler mode transmission signals corresponding to the ensemble number based on the pulse repeat interval.

Referring back to FIG. 3, the ultrasound data acquiring unit 120 further includes a receiving section 330. The receiving section 330 is configured to perform an analog-digital conversion upon the reception signals provided from the ultrasound probe 310 to form sampling data. The receiving section 330 is further configured to perform a reception beam-forming upon the sampling data in consideration of the elements 311 to form reception-focused data. The reception beam-forming will be described below in detail.

In one embodiment, the receiving section 330 is configured to perform the analog-digital conversion upon the brightness mode reception signals provided from the ultrasound probe 310 to form sampling data (hereinafter referred to as "brightness mode sampling data"). The receiving section 330 is further configured to perform the reception beam-forming upon the brightness mode sampling data to form the reception-focused data (hereinafter referred to as "brightness mode reception-focused data").

The receiving section 330 is further configured to perform the analog-digital conversion upon the Doppler mode reception signals provided from the ultrasound probe 310 to form sampling data (hereinafter referred to as "Doppler mode sampling data"). The receiving section 330 is further configured to perform the reception beam-forming upon the Doppler mode sampling data to form reception-focused data (hereinafter referred to as "Doppler mode reception-focused data") corresponding to at least one reception direction of the ultrasound echo signals (i.e., reception beam).

As one example, the receiving section 330 is configured to perform the analog-digital conversion upon the Doppler mode reception signals provided from the ultrasound probe 310 to form the Doppler mode sampling data. The receiving section 330 is further configured to perform the reception beam-forming upon the Doppler mode sampling data to form first Doppler mode reception-focused data corresponding to a first reception direction $Rx_1$ and second Doppler mode reception-focused data corresponding to a second reception direction $Rx_2$, as shown in FIG. 4.

As another example, the receiving section 330 is configured to perform the analog-digital conversion upon the first Doppler mode reception signals provided from the ultrasound probe 310 to form first Doppler mode sampling data corresponding to the first transmission direction $Tx_1$, as shown in FIG. 5. The receiving section 330 is further configured to perform the reception beam-forming upon the first Doppler mode sampling data to form the first Doppler mode reception-focused data corresponding to the first reception direction $Rx_1$. The receiving section 330 is further configured to perform the analog-digital conversion upon the second Doppler mode reception signals provided from the ultrasound probe 310 to form second Doppler mode sampling data corresponding to the second transmission direction $Tx_2$, as shown in FIG. 5. The receiving section 330 is further configured to perform the reception beam-forming upon the second Doppler mode sampling data to form the second Doppler mode reception-focused data corresponding to the second reception direction $Rx_2$. If the reception direction is perpendicular to the elements 311 of the ultrasound probe 310, then a maximum aperture size is used.

The reception beam-forming is described with reference to the accompanying drawings.

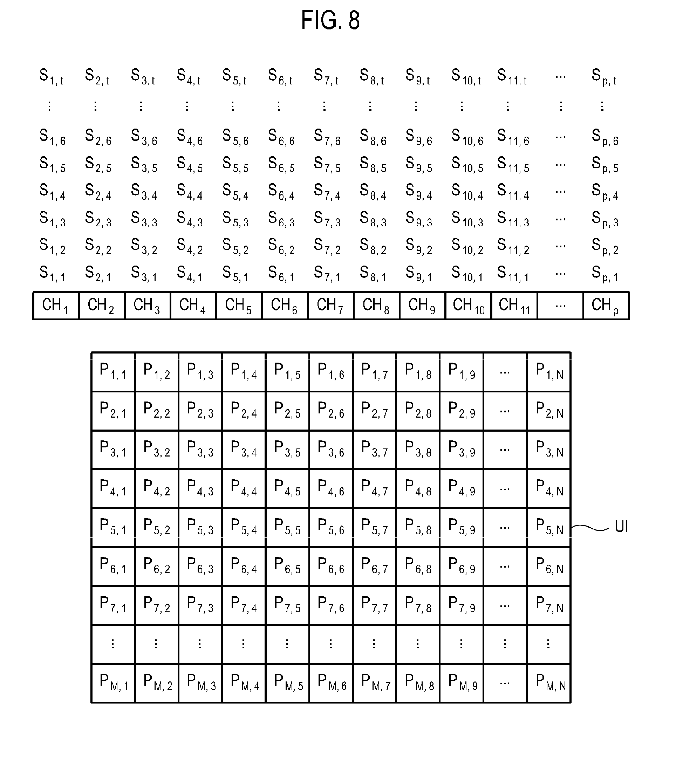

In one embodiment, the receiving section 330 is configured to perform the analog-digital conversion upon the reception signals provided through a plurality of channels $CH_k$, wherein $1 \leq k \leq N$, from the ultrasound probe 310 to form sampling data $S_{i,j}$, wherein the i and j are a positive integer, as shown in FIG. 8. The sampling data $S_{i,j}$ are stored in a storage unit 140. The receiving section 330 is further configured to detect pixels corresponding to the sampling data based on positions of the elements 311 and orientation of pixels of the ultrasound image UI with respect to the elements 311. That is, the receiving section 330 selects the pixels which the respective sampling data are used as pixel data thereof, during the reception beam-forming, based on the positions of the elements 311 and the orientation of the respective pixels of the ultrasound image UI with respect to the elements 311. The receiving section 330 is configured to cumulatively assign the sampling data corresponding to the selected pixels as the pixel data.

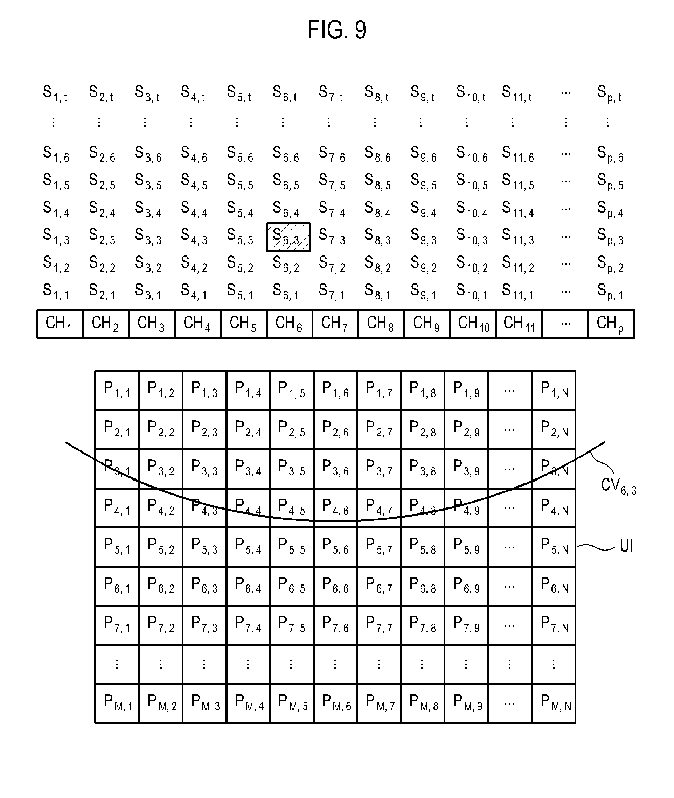

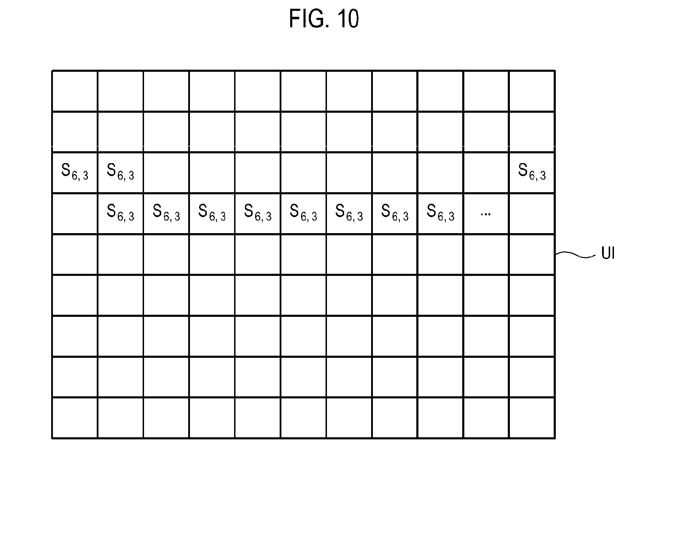

For example, the receiving section 330 is configured to set a curve (hereinafter referred to as "reception beam-forming curve") $CV_{6,3}$ for selecting pixels which the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is further configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10.

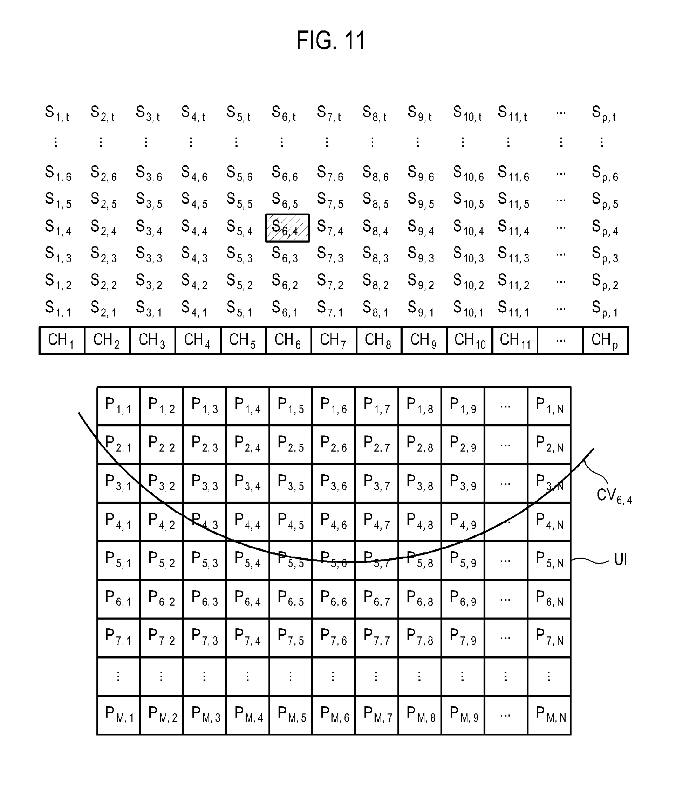

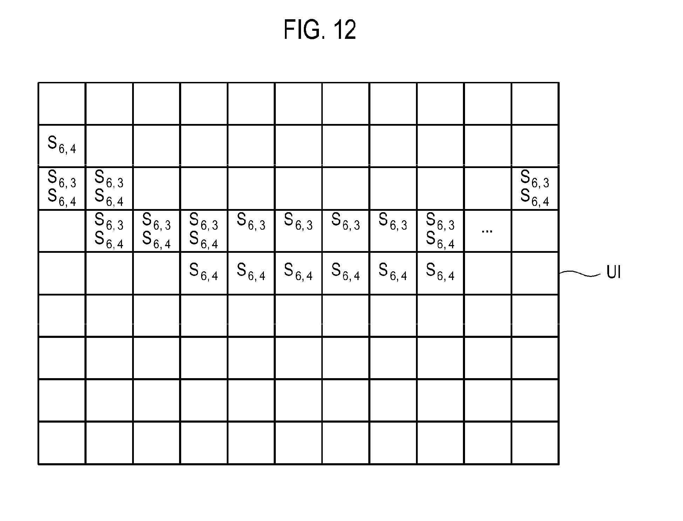

Thereafter, the receiving section 330 is configured to set a reception beam-forming curve $CV_{6,4}$ for selecting pixels which the sampling data $S_{6,4}$ are used as the pixel data thereof, during the reception beam-forming based on the positions of the elements 311 and the orientation of the respective pixels of the ultrasound image UI with respect to the elements 311, as shown in FIG. 11. The receiving section 330 is further configured to detect the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ corresponding to the reception beam-forming curve $CV_{6,4}$ from the pixels $P_{a,b}$ of the ultrasound image UI. That is, the receiving section 330 selects the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ on which the reception beam-forming curve $CV_{6,4}$ passes among the pixels $P_{a,b}$ of the ultrasound image UI. The receiving section 330 is further configured to assign the sampling data $S_{6,4}$ to the selected pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$, as shown in FIG. 12. In this way, the respective sampling data, which are used as the pixel data, are cumulatively assigned to the pixels as the pixel data.

The receiving section 330 is configured to perform the reception beam-forming (i.e., summing) upon the sampling data which are cumulatively assigned to the respective pixels $P_{a,b}$ of the ultrasound image UI to form the reception-focused data.

In another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to detect pixels corresponding to the sampling data based on the positions of the elements 311 and the orientation of the pixels of the ultrasound image UI with respect to the elements 311. That is, the receiving section 330 selects the pixels which the respective sampling data are used as the pixel data thereof, during the reception beam-forming, based on the positions of the elements 311 and the orientation of the respective pixels of the ultrasound image UI with respect to the elements 311. The receiving section 330 is configured to cumulatively assign the sampling data corresponding to the selected pixels as the pixel data. The receiving section 330 is further configured to determine pixels existing in the same column among the selected pixels. The receiving section 330 is further configured to set weights corresponding to the respective determined pixels. The receiving section 330 is further configured to apply the weights to the sampling data of the respective pixels.

For example, the receiving section 330 is configured to set the reception beam-forming curve $CV_{6,3}$ for selecting pixels which the sampling data $S_{6,3}$ are used as the pixel data thereof, during the reception beam-forming, based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI. That is, the receiving section 330 selects the pixels $P_{3,1}$, $P_{3,2}$ $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is further configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ as shown in FIG. 10. The receiving section 330 is further configured to determine pixels $P_{3,2}$ and $P_{4,2}$, which exist in the same column among the selected pixels $P_{3,1}$, $P_{3,2}$, $P_{4,2}$, $P_{4,3}$, $P_{4,4}$,

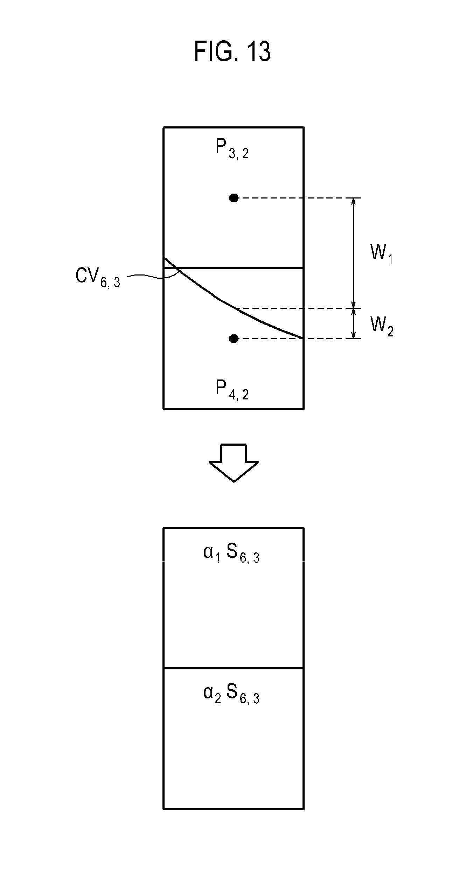

$P_{4,5}$, $P_{4,6}$, $P_{4,7}$, $P_{4,8}$, $P_{4,9}$, ... $P_{3,N}$. The receiving section 330 is further configured to calculate a distance $W_1$ from a center of the determined pixel $P_{3,2}$ to the reception beam-forming curve $CV_{6,3}$ and a distance $W_2$ from a center of the determined pixel $P_{4,2}$ to the reception beam-forming curve $CV_{6,3}$, as shown in FIG. 13. The receiving section 330 is further configured to set a first weight $\alpha_1$ corresponding to the pixel $P_{3,2}$ based on the distance $W_1$ and a second weight $\alpha_2$ corresponding to the pixel $P_{4,2}$ based on the distance $W_2$. The first weight $\alpha_1$ and the second weight $\alpha_2$ are set to be in proportion to or in inverse proportion to the calculated distances. The receiving section 330 is further configured to apply the first weight $\alpha_1$ to the sampling data $S_{6,3}$ assigned to the pixel $P_{3,2}$ and to apply the second weight $\alpha_2$ to the sampling data $S_{6,3}$ assigned to the pixel $P_{4,2}$. The receiving section 330 is configured to perform the above process upon the remaining sampling data.

The receiving section 330 is configured to perform the reception beam-forming upon the sampling data which are cumulatively assigned to the respective pixels $P_{a,b}$ of the ultrasound image UI to form the reception-focused data.

In yet another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to set a sampling data set for selecting pixels which the sampling data $S_{i,j}$ are used as the pixel data thereof, during the reception beam-forming.

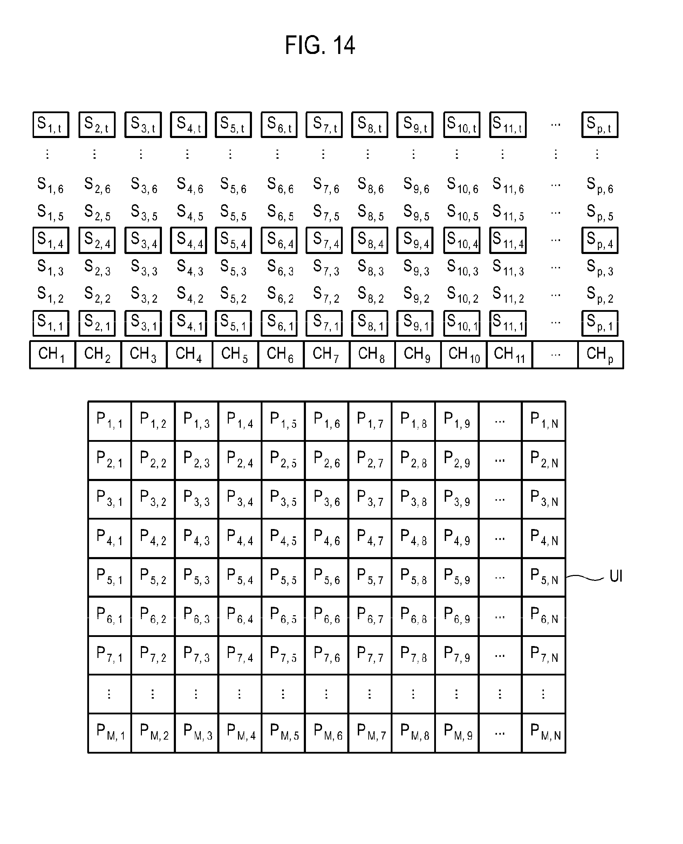

For example, the receiving section 330 is configured to set the sampling data $S_{1,1}$, $S_{1,4}$, ... $S_{1,t}$, $S_{2,1}$, $S_{2,4}$, ... $S_{2,t}$, ... $S_{p,t}$ as the sampling data set (denoted by a box) for selecting the pixels which the sampling data $S_{i,j}$ are used as the pixel data thereof, during the reception beam-forming, as shown in FIG. 14.

The receiving section 330 is further configured to detect the pixels corresponding to the respective sampling data of the sampling data set based on the positions of the elements 311 and the orientation of the respective pixels of the ultrasound image UI with respect to the elements 311. That is, the receiving section 330 selects the pixels which the respective sampling data of the sampling data set are used as the pixel data thereof, during the reception beam-forming, based on the positions of the elements 311 and the orientation of the respective pixels of the ultrasound image UI with respect to the elements 311. The receiving section 330 is further configured to cumulatively assign the sampling data to the selected pixels in the same manner with the above embodiments. The receiving section 330 is further configured to perform the reception beam-forming upon the sampling data which are cumulatively assigned to the respective pixels of the ultrasound image UI to form the reception-focused data.

In yet another embodiment, the receiving section 330 is configured to perform a down-sampling upon the reception signals provided through the plurality of channels $CH_k$ from the ultrasound probe 310 to form down-sampled data. As described above, the receiving section 330 is further configured to detect the pixels corresponding to the respective sampling data, based on the positions of the elements 311 and the orientation of the respective pixels of the ultrasound image UI with respect to the elements 311. That is, the receiving section 330 selects the pixels which the respective sampling data are used as the pixel data thereof, during the reception beam-forming, based on the positions of the elements 311 and the orientation of the pixels of the ultrasound image UI with respect to the elements 311. The receiving section 330 is further configured to cumulatively assign the respective sampling data to the selected pixels in the same manner of the above embodiments. The receiving section 330 is further configured to perform the reception beam-forming upon the sampling data which are cumulatively assigned to the respective pixels of the ultrasound image UI to form the reception-focused data.

However, it should be noted herein that the reception beam-forming may not be limited thereto.

Referring back to FIG. 3, the ultrasound data acquiring unit 120 further includes an ultrasound data forming section 340. The ultrasound data forming section 340 is configured to form the ultrasound data corresponding to the ultrasound image based on the reception-focused data provided from the receiving section 330. The ultrasound data forming section 340 is further configured to perform a signal process (e.g., gain control, etc) upon the reception-focused data.

In one embodiment, the ultrasound data forming section 340 is configured to form the ultrasound data (hereinafter referred to as "brightness mode ultrasound data") corresponding to the brightness mode image based on the brightness mode reception-focused data provided from the receiving section 330. The brightness mode ultrasound data include radio frequency data.

The ultrasound data forming section 340 is further configured to form ultrasound data (hereinafter referred to as "Doppler mode ultrasound data") corresponding to the first region of interest ROI based on the Doppler mode reception-focused data provided from the receiving section 330. The Doppler mode ultrasound data include in-phase/quadrature data. However, it should be noted herein that the Doppler mode ultrasound data may not be limited thereto.

For example, the ultrasound data forming section 340 forms first Doppler mode ultrasound data based on the first Doppler mode reception-focused data provided from the receiving section 330. The ultrasound data forming section 340 further forms second Doppler mode ultrasound data based on the second Doppler mode reception-focused data provided from the receiving section 330.

Referring back to FIG. 1, the ultrasound system 100 further includes a processing unit 130 in communication with the user input unit 110 and the ultrasound data acquiring unit 120. The processing unit 130 includes a central processing unit, a microprocessor, a graphic processing unit and the like.

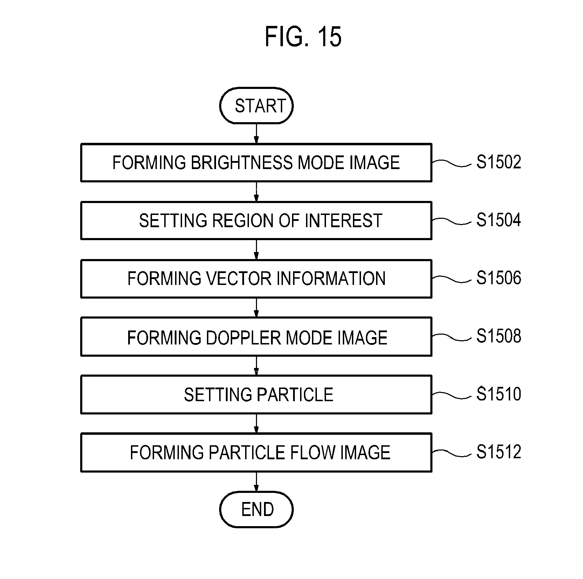

FIG. 15 is a flow chart showing a process of forming a particle flow image. The processing unit 130 is configured to form the brightness mode image BI based on the brightness mode ultrasound data provided from the ultrasound data acquiring unit 120, at step S1502 in FIG. 15. The brightness mode image BI is displayed on a display unit 150.

The processing unit 130 is configured to set the first region of interest ROI on the brightness mode image BI based on the input information (i.e., first input information) provided from the user input unit 110, at step S1504 in FIG. 15. Thus, the ultrasound data acquiring unit 120 is configured to transmit the ultrasound signals to the living body and receive the ultrasound echo signals from the living body to acquire the Doppler mode ultrasound data, in consideration of the first region of interest ROI.

The processing unit 130 is configured to form vector information based on the Doppler mode ultrasound data provided from the ultrasound data acquiring unit 120, at step S1506 in FIG. 15. That is, the processing unit 130 forms the vector information corresponding to motion (i.e., velocity and direction) of the target object based on the Doppler mode ultrasound data.

Generally, when the transmission direction of the ultrasound signals is equal to the reception direction of the ultrasound echo signals and a Doppler angle is $\theta$, the following relationship is established:

$$X \cos \theta = \frac{C_0 f_d}{2 f_0} \qquad (1)$$

In equation 1, X represents a reflector velocity (i.e., velocity of target object), $C_0$ represents a sound speed in the living body, $f_d$ represents a Doppler shift frequency, and $f_0$ represents an ultrasound frequency.

The Doppler shift frequency $f_d$ is calculated by the difference between a frequency of the ultrasound signals (i.e., transmission beam) and a frequency of the ultrasound echo signals (i.e., reception beam). Also, the velocity component $X\cos\theta$ projected to the transmission direction is calculated by equation 1.

When the transmission direction of the ultrasound signals (i.e., transmission beam) is different to the reception direction of the ultrasound echo signals (i.e., reception beam), the following relationship is established:

$$X \cos \theta_T + X \cos \theta_R = \frac{C_0 f_d}{f_0} \qquad (2)$$

In equation 2, $\theta_T$ represents an angle between the ultrasound signals (i.e., transmission beam) and the blood flow, and $\theta_R$ represents an angle between the ultrasound echo signals (i.e., reception beam) and the blood flow.

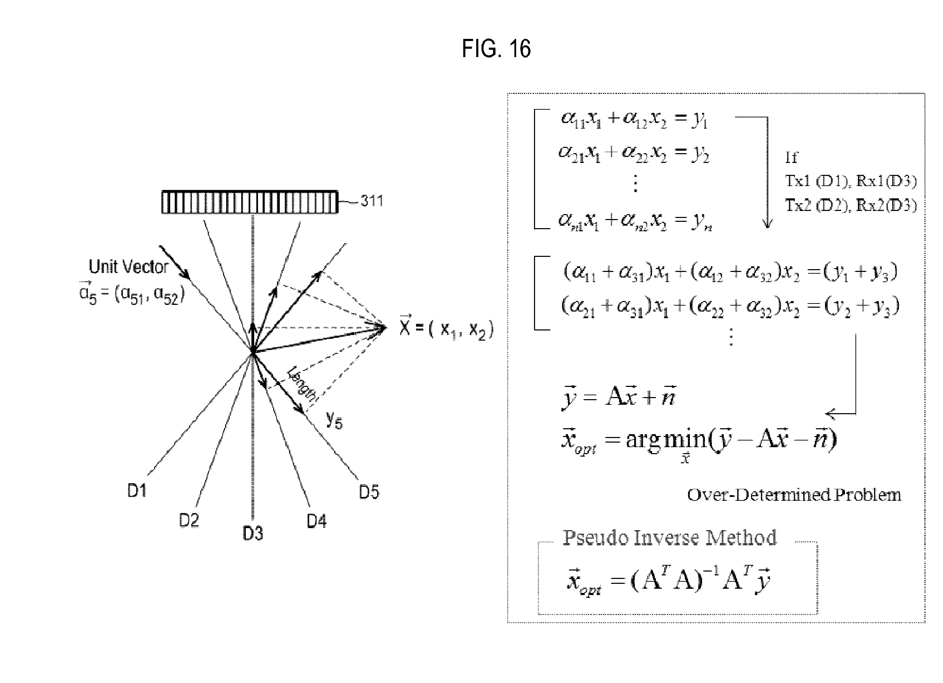

FIG. 16 is a schematic diagram showing an example of the transmission directions, the reception directions, the vector information and an over-determined problem. Referring to FIG. 16, when the ultrasound signals (i.e., transmission beam) are transmitted in a first direction D1 and the ultrasound echo signals (i.e., reception beam) are received in the first direction D1, the following relationship is established:

$$\vec{\alpha_1}\vec{X} = \alpha_{11}x_1 + \alpha_{12}x_2 = y_1 = X \cos \theta \qquad (3)$$

In equation 3, $\vec{\alpha_1}=(\alpha_{11},\alpha_{12})$ represents a unit vector of the first direction D1, $\vec{X} = (x_1,x_2)$ represents variables, and $y_1$ is calculated by equation 1.

When the ultrasound signals (i.e., transmission beam) are transmitted in a second direction D2 and the ultrasound echo signals (i.e., reception beam) are received in a third direction D3, the following relationship is established:

$$(\alpha_{21}+\alpha_{31})x_1 + (\alpha_{22}+\alpha_{32})x_2 = (y_2 + y_3) = X \cos \theta_2 + X \cos \theta_3 \qquad (4)$$

Equations 3 and 4 assume two-dimensional environment, equations 3 and 4 are expanded to three-dimensional environment. That is, when expanding equations 3 and 4 to the three-dimensional environment, the following relationship

is established:

$$\alpha_{11}x_1 + \alpha_{12}x_2 + \alpha_{13}x_3 = y \qquad\qquad (5)$$

In the case of the two-dimensional environment (i.e., two-dimensional vector), at least two equations are required to calculate the variables $x_1$ and $x_2$. For example, when the ultrasound signals (i.e., transmission beam) are transmitted in the third direction D3 and the ultrasound echo signals (i.e., reception beam) are received in the second direction D2 and a fourth direction D4 as shown in FIG. 16, the following equations are established:

$$(\alpha_{31} + \alpha_{21})x_1 + (\alpha_{32} + \alpha_{22})x_2 = (y_3 + y_2)$$

$$(\alpha_{31} + \alpha_{41})x_1 + (\alpha_{32} + \alpha_{42})x_2 = (y_3 + y_4) \qquad\qquad (6)$$

The vector $\vec{X} = (x_1, x_2)$ is calculated by the equations of equation 6.

When the reception beam-forming is performed in at least two angles (i.e., at least two reception directions), at least two equations are obtained and represented as the over-determined problem, as shown in FIG. 16. The over-determined problem is solved by a pseudo inverse method, a weighted least square method and the like based on noise characteristics added to the Doppler shift frequency. That is, M×N equations are obtained by M transmission directions and the reception beam-forming of N reception directions at every transmission.

Referring back to FIG. 15, the processing unit 130 is configured to form the Doppler mode image based on the vector information, at step S1508 in FIG. 15. The Doppler mode image is displayed on the display unit 150. Thus, the user sets the at least one second region of interest on the Doppler mode image displayed on the display unit 150, and sets the weighting value corresponding to the at least one second region of interest.

The processing unit 130 is configured to set the at least one second region of interest on the Doppler mode image based on the input information (i.e., second input information) provided from the user input unit 110, at step S1510 in FIG. 15. For example, the processing unit130 sets the second region of interest PT on the Doppler mode image VDI based on the input information (i.e., second input information).

The processing unit 130 is configured to form the particle flow image for representing flow of the at least one second region of interest, which is set on the Doppler mode image, based on the vector information and the input information (i.e., third input information) provided from the user input unit 110, at step S 1512 in FIG. 15.

In the embodiment, the processing unit 130 is configured to determine a movement direction and velocity corresponding to the second region of interest PT by a predetermined time based on the vector information including a vector direction and a vector magnitude corresponding to the motion (i.e., velocity and direction) of the target object (e.g., blood flow). The methods of determining the movement direction and the velocity corresponding to the second region of interest PT based on the vector information are well known in the art. Thus, they have not been described in detail so as not to unnecessarily obscure the present disclosure. The processing unit 130 is further configured to apply the weighting value to the determined velocity, based on the input information (i.e., third input information) provided from the user input unit 110 to calculate an actual velocity corresponding to the second region of interest. The processing unit 130 is further configured to form the particle flow image for representing the flow of the second region of interest based on the movement direction and the actual velocity corresponding to the second region of interest.

As one example, the processing unit 130 forms the particle flow image $PTI_1$ for representing the flow of the second region of interest PT as points based on the movement direction and the actual velocity corresponding to the second region of interest PT.

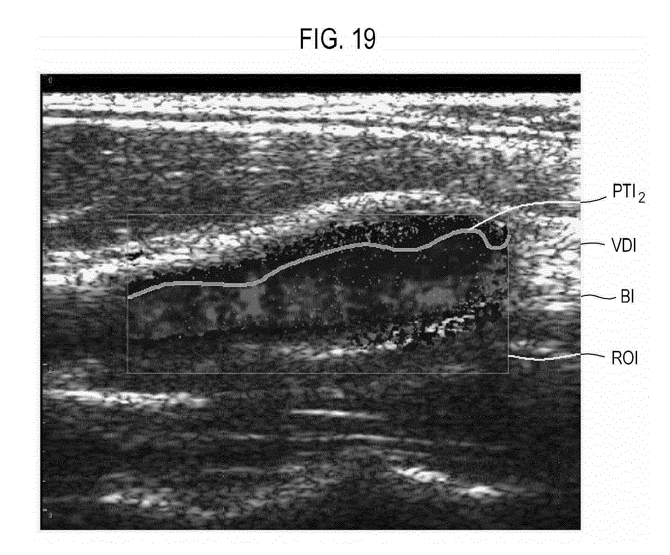

As another example, the processing unit 130 forms the particle flow image $PTI_2$ for representing the flow of the second region of interest as a stream line based on the movement direction and the actual velocity corresponding to the second region of interest PT, as shown in FIG. 19. That is, the processing unit 130 moves the second region of interest PT by the predetermined time based on the vector information. The processing unit 130 moves the second region of interest PT until the second region of interest PT gets out of the Doppler mode image VDI or until the vector information does not exist. The processing unit 130 further connects points moved by the second region of interest PT to form the stream line. That is, the processing unit 130 forms a trajectory of the points moved by the second region of interest PT to form the stream line.

Referring back to FIG. 1, the ultrasound system 100 further includes the storage unit 140. The storage unit 140 stores the ultrasound data (i.e., brightness mode ultrasound data and Doppler mode ultrasound data) acquired by the ultrasound data acquiring unit 120. The storage unit 140 further stores the vector information formed by the processing unit 130.

The ultrasound system 100 further includes the display unit 150. The display unit 150 is configured to display the brightness mode image BI formed by the processing unit 130. The display unit 150 is further configured to display the Doppler mode image VDI formed by the processing unit 130. The display unit 150 is further configured to display the particle flow image $PT_1$ or $PT_2$ formed by the processing unit 130.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1.  An ultrasound system, comprising:

    a processing unit configured to form vector information of a target object based on ultrasound data corresponding to the target object, form a Doppler mode image based on the vector information, and set at least one region of interest on the Doppler mode image based on input information of a user, the processing unit being further configured to form a flow image for representing flow of the at least one region of interest based on the vector information.

2.  The ultrasound system of Claim 1, wherein the region of interest includes a particle.

3.  The ultrasound system of Claim 1, wherein the processing unit is configured to form the vector information corresponding to a velocity and a direction of the target object in consideration of at least one transmission direction and at least one reception direction corresponding to the at least one transmission direction.

4.  The ultrasound system of Claim 1, further comprising:

    a user input unit configured to receive the input information for setting the at least one region of interest on the Doppler mode image and setting a weighting value corresponding to the at least one region of interest.

5.  The ultrasound system of Claim 4, wherein the processing unit is configured to:

    determine a movement direction and a velocity corresponding to the at least one region of interest by a predetermined time based on the vector information;
    apply the weighting value to the determined velocity based on the input information to calculate an actual velocity corresponding to the at least one region of interest; and
    form the particle flow image based on the movement direction and the actual velocity corresponding to the at least one region of interest.

6.  The ultrasound system of Claim 5, wherein the processing unit is configured to form the particle flow image for representing the flow of the at least one region of interest as points based on the movement direction and the actual velocity corresponding to the at least one region of interest.

7.  The ultrasound system of Claim 5, wherein the processing unit is configured to form the particle flow image for representing the flow of the at least one region of interest as a stream line based on the movement direction and the actual velocity corresponding to the at least one region of interest.

8.  The ultrasound system of Claim 1, further comprising:

    an ultrasound data acquiring unit configured to transmit ultrasound signals to a living body including the target object in at least one transmission direction, and receive ultrasound echo signals from the living body in at least one reception direction to acquire the ultrasound data corresponding to the at least one reception direction.

9. A method of providing a particle flow image, comprising:

a) forming vector information of a target object based on ultrasound data corresponding to the target object;
b) forming a Doppler mode image based on the vector information;
c) setting at least one region of interest on the Doppler mode image based on input information of a user; and
d) forming a flow image for representing flow of the at least one region of interest based on the vector information.

10. The method of Claim 9, wherein the region of interest includes a particle.

11. The method of Claim 9, wherein the step b) comprises:

forming the vector information corresponding to a velocity and a direction of the target object in consideration of at least one transmission direction and at least one reception direction corresponding to the at least one transmission direction.

12. The method of Claim 9, further comprising:

receiving the input information for setting the at least one region of interest on the Doppler mode image and for setting a weighting value corresponding to the at least one region of interest, prior to performing the step c).

13. The method of Claim 12, wherein the step d) comprises:

d1) determining a movement direction and a velocity corresponding to the at least one region of interest by a predetermined time based on the vector information;
d2) applying the weighting value to the determined velocity based on the input information to calculate an actual velocity corresponding to the at least one region of interest; and
d3) forming the particle flow image based on the movement direction and the actual velocity corresponding to the at least one region of interest.

14. The method of Claim 13, wherein the step d3) comprises:

forming the particle flow image for representing the flow of the at least one region of interest as points based on the movement direction and the actual velocity corresponding to the at least one region of interest.

15. The method of Claim 13, wherein the step d3) comprises:

forming the particle flow image for representing the flow of the at least one region of interest as a stream line based on the movement direction and the actual velocity corresponding to the at least one region of interest.

16. The method of Claim 9, further comprising:

transmitting ultrasound signals to a living body including the target object in at least one transmission direction and receiving ultrasound echo signals from the living body in at least one reception direction to acquire the ultrasound data corresponding to the at least one reception direction, prior to performing the step a).

# FIG. 1

100

110

USER
INPUT UNIT

120

ULTRASOUND
DATA ACQUIRING
UNIT

130

PROCESSING
UNIT

150

DISPLAY
UNIT

STORAGE
UNIT 140

# FIG. 2

# FIG. 3

# FIG. 4

311

Rx₂

Rx₁

Tx

## FIG. 5

311

Rx$_1$, Rx$_2$

Tx$_1$

Tx$_2$

Tx$_1$ (ensemble number)

Tx$_2$ (ensemble number)

PRI

...

...

time

# FIG. 6

311

Rx$_1$, Rx$_2$

Tx$_1$          Tx$_2$

Tx$_1$  Tx$_2$      Tx$_1$  Tx$_2$      Tx$_1$  Tx$_2$   ...

PRI

time

# FIG. 7

311

Rx$_1$          Rx$_2$

Tx$_1$          Tx$_2$

Tx$_1$   PRI   Tx$_2$   PRI   Tx$_1$   PRI   Tx$_2$   ...

time

# FIG. 8

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

UI

# FIG. 9

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,3}$

UI

FIG. 10

| | | | | | | | | | | |
|---|---|---|---|---|---|---|---|---|---|---|
| | | | | | | | | | | |
| | | | | | | | | | | |
| $S_{6,3}$ | $S_{6,3}$ | | | | | | | | | $S_{6,3}$ |
| | $S_{6,3}$ | $S_{6,3}$ | $S_{6,3}$ | $S_{6,3}$ | $S_{6,3}$ | $S_{6,3}$ | $S_{6,3}$ | $S_{6,3}$ | ... | |
| | | | | | | | | | | |
| | | | | | | | | | | |
| | | | | | | | | | | |
| | | | | | | | | | | |
| | | | | | | | | | | |

UI

# FIG. 11

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,4}$

$UI$

## FIG. 12

|  | | | | | | | | | | | |
|---|---|---|---|---|---|---|---|---|---|---|---|
| $S_{6,4}$ | | | | | | | | | | | |
| $S_{6,3}$ $S_{6,4}$ | $S_{6,3}$ $S_{6,4}$ | | | | | | | | | $S_{6,3}$ $S_{6,4}$ | |
| | $S_{6,3}$ $S_{6,4}$ | $S_{6,3}$ $S_{6,4}$ | $S_{6,3}$ $S_{6,4}$ | $S_{6,3}$ | $S_{6,3}$ | $S_{6,3}$ | $S_{6,3}$ | $S_{6,3}$ $S_{6,4}$ | ... | | |
| | | | $S_{6,4}$ | $S_{6,4}$ | $S_{6,4}$ | $S_{6,4}$ | $S_{6,4}$ | $S_{6,4}$ | | | |
| | | | | | | | | | | | |
| | | | | | | | | | | | |
| | | | | | | | | | | | |
| | | | | | | | | | | | |

UI

## FIG. 13

$P_{3,2}$

$CV_{6,3}$

$W_1$

$W_2$

$P_{4,2}$

$\alpha_1 S_{6,3}$

$\alpha_2 S_{6,3}$

## FIG. 14

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | ... | $S_{p,t}$ |
|---|---|---|---|---|---|---|---|---|---|---|---|---|
| ⋮ | ⋮ | ⋮ | ⋮ | ⋮ | ⋮ | ⋮ | ⋮ | ⋮ | ⋮ | ⋮ | ⋮ | ⋮ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | ... | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | ... | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | ... | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | ... | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | ... | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | ... | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | ... | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | ... | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | ... | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | ... | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | ... | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | ... | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | ... | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | ... | $P_{7,N}$ |
| ⋮ | ⋮ | ⋮ | ⋮ | ⋮ | ⋮ | ⋮ | ⋮ | ⋮ | ⋮ | ⋮ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | ... | $P_{M,N}$ |

UI

# FIG. 15

```
START
  │
  ▼
FORMING BRIGHTNESS MODE IMAGE     ⟋ S1502
  │
  ▼
SETTING REGION OF INTEREST        ⟋ S1504
  │
  ▼
FORMING VECTOR INFORMATION        ⟋ S1506
  │
  ▼
FORMING DOPPLER MODE IMAGE        ⟋ S1508
  │
  ▼
SETTING PARTICLE                  ⟋ S1510
  │
  ▼
FORMING PARTICLE FLOW IMAGE       ⟋ S1512
  │
  ▼
END
```

# FIG. 16

EP 2 609 869 A1

$$\begin{bmatrix} \alpha_{11}x_1 + \alpha_{12}x_2 = y_1 \\ \alpha_{21}x_1 + \alpha_{22}x_2 = y_2 \\ \vdots \\ \alpha_{n1}x_1 + \alpha_{n2}x_2 = y_n \end{bmatrix}$$

If
Tx1 (D1), Rx1(D3)
Tx2 (D2), Rx2(D3)

$$\begin{bmatrix} (\alpha_{11} + \alpha_{31})x_1 + (\alpha_{12} + \alpha_{32})x_2 = (y_1 + y_3) \\ (\alpha_{21} + \alpha_{31})x_1 + (\alpha_{22} + \alpha_{32})x_2 = (y_2 + y_3) \\ \vdots \end{bmatrix}$$

$$\vec{y} = A\vec{x} + \vec{n}$$

$$\vec{x}_{opt} = \arg\min_{\vec{x}}(\vec{y} - A\vec{x} - \vec{n})$$

Over-Determined Problem

Pseudo Inverse Method

$$\vec{x}_{opt} = (A^T A)^{-1} A^T \vec{y}$$

Unit Vector
$\vec{a}_5 = (a_{51}, a_{52})$

$\vec{X} = (x_1, x_2)$

Length

$y_5$

311

D1  D2  D3  D4  D5

FIG. 17

## FIG. 18

# FIG. 19

Europäisches
Patentamt

European
Patent Office

Office européen
des brevets

# EUROPEAN SEARCH REPORT

Application Number

EP 12 19 9547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 931 784 A (KAJIWARA SOUICHI [JP] ET AL) 3 August 1999 (1999-08-03) | 1-3, 8-11,16 | INV. A61B8/06 |
| Y | * column 6, line 33 - line 40 * <br> * column 8, line 27 - line 50 * <br> ----- | 4-7, 12-15 | G01S15/89 |
| X | US 5 910 119 A (LIN GREGORY SHARAT [US]) 8 June 1999 (1999-06-08) <br> * column 7, line 66 - column 8, line 5 * <br> * column 9, line 66 - column 10, line 9 * <br> * column 10, line 28 - line 38 * <br> ----- | 1-3, 8-11,16 | |
| X | SCABIA M ET AL: "A real-time two-dimensional pulsed-wave Doppler system", <br> ULTRASOUND IN MEDICINE AND BIOLOGY, NEW YORK, NY, US, <br> vol. 26, no. 1, <br> 1 January 2000 (2000-01-01), pages 121-131, XP004295497, <br> ISSN: 0301-5629, DOI: 10.1016/S0301-5629(99)00115-5 <br> * page 126, column 1, line 6 - line 19; figure 5 * <br> ----- | 1-3, 8-11,16 | |
| Y | US 4 142 412 A (MCLEOD FRANCIS ET AL) 6 March 1979 (1979-03-06) <br> * claim 11 * <br> ----- | 4-7, 12-15 | TECHNICAL FIELDS SEARCHED (IPC) <br><br> A61B <br> G01S |

The present search report has been drawn up for all claims

| Place of search | Date of completion of the search | Examiner |
|---|---|---|
| The Hague | 8 March 2013 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 19 9547

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2013

| Patent document cited in search report | | Publication date | Patent family member(s) | | Publication date |
|---|---|---|---|---|---|
| US 5931784 | A | 03-08-1999 | CN | 1182357 A | 20-05-1998 |
| | | | EP | 0830842 A1 | 25-03-1998 |
| | | | US | 5931784 A | 03-08-1999 |
| | | | WO | 9734530 A1 | 25-09-1997 |
| US 5910119 | A | 08-06-1999 | EP | 0957374 A2 | 17-11-1999 |
| | | | JP | 4297555 B2 | 15-07-2009 |
| | | | JP | 2000201931 A | 25-07-2000 |
| | | | US | 5910119 A | 08-06-1999 |
| US 4142412 | A | 06-03-1979 | AU | 2130977 A | 20-07-1978 |
| | | | CA | 1112351 A1 | 10-11-1981 |
| | | | DE | 2703486 A1 | 01-12-1977 |
| | | | ES | 455269 A1 | 01-01-1978 |
| | | | FR | 2351391 A1 | 09-12-1977 |
| | | | FR | 2356126 A1 | 20-01-1978 |
| | | | FR | 2356127 A1 | 20-01-1978 |
| | | | FR | 2356143 A1 | 20-01-1978 |
| | | | GB | 1587151 A | 01-04-1981 |
| | | | GB | 1587152 A | 01-04-1981 |
| | | | IT | 1212224 B | 22-11-1989 |
| | | | JP | 52137379 A | 16-11-1977 |
| | | | US | 4142412 A | 06-03-1979 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110144461